# EUROPEAN PATENT APPLICATION

(11) **EP 4 353 738 A1**
(43) Date of publication of application: **17.04.2024**
(21) Application number: 21945282.8
(22) Date of filing: 06.09.2021
(51) Int. Cl.: C07K 14/245, C12N 15/70, C12P 13/06, C12P 13/12

(54) **NOVEL MDTH VARIANT AND METHOD FOR PRODUCING O-PHOSPHOSERINE, CYSTEINE, AND DERIVATE OF CYSTEINE USING SAME**

(30) Priority: 11.06.2021 KR 20210075859
(71) Applicant: CJ CHEILJEDANG CORPORATION, Seoul 04560 (KR)
(72) Inventor: SIM, Hee-jin, Seoul 04560 (KR); PARK, Hye Min, Seoul 04560 (KR); CHOI, Jin-Geun, Seoul 04560 (KR); LEE, Jin Nam, Seoul 04560 (KR); JUNG, Hwi-Min, Seoul 04560 (KR)
(74) Representative: Icosa
(86) International application number: PCT/KR2021/011995
(87) International publication number: WO 2022/260215

(57) **Abstract**

The present disclosure relates to a novel MdtH variant and methods for producing O-phosphoserine and cysteine and a derivative of cysteine by using the same.

## Description

### [Technical Field]

The present disclosure relates to a MdtH variant and methods for producing O-phosphoserine and cysteine and a derivative of cysteine by using the same.

### [Background Art]

L-Cysteine, which is an important amino acid in sulfur metabolism of all living organisms, is used not only in the synthesis of *in vivo* proteins such as hair keratin, glutathione, biotin, methionine, and other sulfur-containing metabolites, but also as a precursor in the biosynthesis of coenzyme A.

As for the production of L-cysteine through the use of microorganisms, disclosed are: 1) a method of biologically converting D,L-2-aminothiazoline-4-carboxylic acid (D,L-ATC) by using microorganisms; 2) a direct fermentation method of producing L-cysteine by using *E*. *coli* (European Patent EP 0885962 B; and Wada M and Takagi H, Appl. Microbiol. Biochem., 73:48-54, 2006); and 3) a method of producing *O*-phosphoserine (hereinafter, "OPS") by fermentation using microorganisms and then converting OPS into L-cysteine by reacting OPS with a sulfide under the catalytic action of *O*-phosphoserine sulfhydrylase (hereinafter, "OPSS") (European Patent Publication EP 2444481).

However, research on effective L-amino acid production methods is still needed due to the increasing demand for L-cysteine. In particular, for the highyield production of cysteine by way of method 3), there was a need to overproduce the precursor OPS.

### [Disclosure]

### [Technical Problem]

As a result of intensive efforts to overproduce its precursor, OPS, in order to produce high yield of cysteine, the present inventors have completed this application by confirming that a MdtH variant increases OPS-producing ability.

### [Technical Solution]

An aspect of the present disclosure is to provide a MdtH variant, in which the amino acid corresponding to position 125 in the amino acid sequence of SEQ ID NO: 1 is substituted with another amino acid.

Another aspect of the present disclosure is to provide a polynucleotide encoding the variant of the present disclosure.

Still another aspect of the present disclosure is to provide a vector containing the polynucleotide of the present disclosure.

Still another aspect of the present disclosure is to provide a recombinant microorganism of the genus *Escherichia,* containing a MdtH variant, in which the amino acid corresponding to position 125 in the amino acid sequence of SEQ ID NO: 1 is substituted with another amino acid, or a polynucleotide encoding the variant.

Still another aspect of the present disclosure is to provide a method for producing O-phosphoserine, the method including culturing in a medium a microorganism containing a MdtH variant, in which the amino acid corresponding to position 125 in the amino acid sequence of SEQ ID NO: 1 is substituted with another amino acid, or a polynucleotide encoding the variant.

Still another aspect of the present disclosure is to provide a method for producing cysteine or a derivative thereof, the method including: a) culturing in a medium a microorganism containing a MdtH variant, in which the amino acid corresponding to position 125 in the amino acid sequence of SEQ ID NO: 1 is substituted with another amino acid, or a polynucleotide encoding the variant, to thereby produce *O*-phosphoserine or a medium containing the *O*-phosphoserine; and b) reacting a sulfide with *O*-phosphoserine sulfhydrylase (OPSS) or a microorganism expressing OPSS and *O*-phosphoserine or the medium containing *O*-phosphoserine produced in step a).

### [Advantageous Effects]

The culture of a microorganism having the OPS producing ability by using the novel variant polypeptide having an OPS export activity of the present disclosure can produce OPS with a high yield compared with the use of an existing non-modified or variant protein.

### [Detailed Description of Preferred Embodiments]

A specific description will be made as follows. Each description and embodiment disclosed in this disclosure may also be applied to other descriptions and embodiments. That is, all combinations of various elements disclosed in this disclosure fall within the scope of the present disclosure. Furthermore, the scope of the present disclosure is not limited by the specific description below.

In accordance with one aspect of the present disclosure, there is provided a MdtH variant, in which the amino acid corresponding to position 125 in the amino acid sequence of SEQ ID NO: 1 is substituted with another amino acid.

In an embodiment, the other amino acid may be isoleucine.

The variant of the present disclosure may be a variant in which valine, the amino acid corresponding to position 125 in the amino acid sequence set forth in SEQ ID NO: 1 as a parent sequence, is substituted with isoleucine, and which has homology or identity of at least 70%, at least 75%, at least 80%, at least 85%, at least 90%, at least 95%, at least 96%, at least 97%, at least 98%, at least 99%, at least 99.5%, or at least 99.7%, and less than 100% with the amino acid sequence set forth in SEQ ID NO: 1. For example, the variant of the present disclosure may have isoleucine as the amino acid corresponding to position 125 in the amino acid sequence set forth in SEQ ID NO: 1, and may have or contain an amino acid sequence having homology or identity of at least 70%, at least 75%, at least 80%, at least 85%, at least 90%, at least 95%, at least 96%, at least 97%, at least 98%, at least 99%, at least 99.5%, or at least 99.7%, and less than 100% with the amino acid sequence set forth in SEQ ID NO: 1, or may consist of or consist essentially of the amino acid sequence. It is also obvious that even a variant having an amino acid sequence with a deletion, modification, substitution, conservative substitution, or addition in a part thereof may also fall within the scope of the present disclosure as long as the amino acid sequence has such homology or identity and exhibits activity corresponding to the variant of the present disclosure.

For example, there may be a sequence addition or deletion, a naturally occurring mutation, a silent mutation, or a conservative substitution, which does not alter functions of the variant of the present disclosure, in the N-terminus, C-terminus, and/or inside of the amino acid sequence.

The "conservative substitution" refers to a substitution of one amino acid with another amino acid having similar structural and/or chemical properties thereto. Such an amino acid substitution may generally occur on the basis of similarities in polarity, charge, solubility, hydrophobicity, hydrophilicity, and/or amphipathic nature of residues. Usually, the conservative substitution may have little or no effect on the activity of a protein or a polypeptide.

As used herein, the term "variant" refers to a particular protein that has a different sequence from the parent sequence thereof due to a conservative substitution and/or modification of at least one amino acid in the parent sequence but has unchanged functions or properties. A variant differs from a sequence identified by several amino acid substitutions, deletions, or additions. This variant can be generally identified by modifying at least one amino acid in the amino acid sequence of the particular protein and evaluating the properties of the modified protein. That is, the ability of a variant may be increased, unchanged, or decreased compared with that of its native protein. In addition, some variants may include variants in which at least one part, such as a N-terminal leader sequence or a transmembrane domain, is removed. Other variants may include variants in which a part of the N-terminus and/or C-terminus of a mature protein is removed. The term "variant" may also be used interchangeably with "modification", "modified protein", "modified polypeptide", "mutant", "mutein", "divergent", or the like, and any term that is used in a sense of being modified can be used without limitation thereto. For the purpose of the present disclosure, the variant may refer to a variant in which the activity of a modified protein is increased compared with a naturally occurring wild-type or non-modified protein, but the variant is not limited thereto.

The variant may also contain deletions or additions of amino acids having minimal influence on the properties and secondary structure of a polypeptide. For example, a signal (or leader) sequence involved in co-translational or posttranslational protein translocation may be conjugated to the N-terminus of the variant. In addition, the variant may also be conjugated to another sequence or a linker for identification, purification, or synthesis thereof.

As used herein, the term "parent sequence" refers to a reference sequence, which becomes a modified polypeptide through the introduction of modification. That is, the parent sequence, which is a starting sequence, may be an object into which a modification, such as a substitution, an insertion, and/or a deletion, is introduced. The parent sequence may be in a naturally occurring type or a wild type, and may be a variant having at least one substitution, insertion, or deletion occurring in the naturally occurring type or wild type or an artificially synthesized sequence.

As used herein, the term "homology" or "identity" refers to the degree of similarity between two given amino acid sequences or nucleotide sequences, and may be expressed as a percentage. The terms homology and identity may often be used interchangeably.

The sequence homology or identity of conserved polynucleotides or polypeptides may be determined using a standard alignment algorithm, and default gap penalties established by a program to be used may be used in combination. Substantially, homologous or identical sequences may generally hybridize, under moderate or highly stringent conditions, with the entire sequence or a part of it. It is obvious that the hybridization also includes a hybridization with a polynucleotide containing a typical codon or a codon chosen in consideration of codon degeneracy.

It may be determined whether any two polynucleotide or polypeptide sequences have homology, similarity, or identity, by using a known computer algorithm, such as the "FASTA" program, through the use of default parameters, as in Pearson et al., (1988) Proc. Natl. Acad. Sci. USA 85:2444. Alternatively, the homology, similarity, or identity may be determined using the Needleman-Wunsch algorithm (Needleman and Wunsch, 1970, J. Mol. Biol. 48:443-453), as implemented in the Needleman program (version 5.0.0 or versions thereafter) of the European Molecular Biology Open Software Suite (EMBOSS) package (Rice et al., 2000, Trends Genet. 16:276-277) (including GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be determined by comparing sequence information through the GAP computer program, for example, Needleman et al., (1970), J Mol Biol. 48:443, as known in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of symbols in the shorter of the two sequences. The default parameters for the GAP program may include: (1) a binary comparison matrix (containing a value of 1 for identity and 0 for non-identity) and a weighted comparison matrix of Gribskov et al (1986) Nucl. Acids Res. 14:6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10 and a gap extension penalty of 0.5); and (3) no penalty for end gaps.

As used herein, the term "corresponding to" refers to an amino acid residue at a position recited in a polypeptide, or an amino acid residue similar, identical, or homologous to the residue recited in the polypeptide. Identifying an amino acid at a corresponding position may be determining a particular amino acid of a sequence referring to a particular sequence. As used herein, the term "corresponding region" generally refers to a similar or corresponding position in a related protein or reference protein.

For example, any amino acid sequence is aligned with SEQ ID NO: 1, and based on this, each amino acid residue of the amino acid sequence may be numbered with reference to an amino acid residue numerical position corresponding to an amino acid residue in SEQ ID NO: 1. For example, a sequence alignment algorithm as described in the present disclosure may identify the position of an amino acid or a position of occurrence of a modification, such as a substitution, an insertion, or a deletion, compared with a query sequence (also referred to as a "reference sequence").

For example, the Needleman-Wunsch algorithm (Needleman and Wunsch, 1970, J. Mol. Biol. 48:443-453), the Needle program of the EMBOSS package (EMBOSS: The European Molecular Biology Open Software Suite, Rice et al., 2000, Trends Genet. 16:276-277), or the like may be used for such alignment, but without limitation thereto, a sequence alignment program, a pairwise sequence comparison algorithm, or the like, which is known in the art, may be used as appropriate.

As used herein, the term "MdtH" refers to a kind of transports of the major facilitator superfamily (MFS), which is a superfamily of membrane transport proteins that facilitate movement of small solutes across cell membranes in response to chemiosmotic gradients, and which is known as a protein that exhibits an OPS export activity from *E. coli* where growth inhibition is released in the presence of excessive OPS. The MdtH herein may mean a membrane protein having an activity to export *O*-phosphoserine (OPS) out of cells. The sequence of the MdtH may be obtained from a known database, GenBank of NCBI. For example, the MdtH may be a polypeptide having an OPS export activity, encoded by mdtH gene, but is not limited thereto.

The variant of the present disclosure may have an activity to increase the OPS export ability compared with a wild-type polypeptide.

As used herein, the term "O-phosphoserine (hereinafter, "OPS")" refers to a phosphoric acid ester of serine, which is a constituent component for several proteins. The OPS, which is a precursor of L-cysteine, may be converted into cysteine by reaction with a sulfide under the catalytic action of OPS sulfhydrylase (OPSS), but is not limited thereto (U.S. Patent Application Publication US 2012-0190081).

The variant of the present disclosure may be one in which at least one of the amino acids corresponding to positions 60, 180, and 398 in the amino acid sequence of SEQ ID NO: 1 is further substituted with another amino acid. For example, the variant of the present disclosure may be one in which an amino acid corresponding to position 125 in the amino acid sequence of SEQ ID NO: 1 is substituted with another amino acid and at least one, at least two, or at least three of the amino acids corresponding to positions 60, 180, and 398 are further substituted with other amino acids, but is not limited thereto.

Specifically, the variant of the present disclosure may be a modified polypeptide that exhibits an OPS export activity, in which, from the N-terminus of the amino acid sequence of SEQ ID NO: 1, i) valine, the amino acid residue corresponding to position 125, is substituted with an amino acid residue other than valine; or further, ii) glutamine, the amino acid residue corresponding to position 60, iii) phenylalanine, the amino acid residue corresponding to position 180, and/or iv) leucine, the amino acid residue corresponding to position 398, are each substituted with other amino acid residues.

From the N-terminus of the amino acid sequence of SEQ ID NO: 1, the amino acid other than i) valine, the amino acid residue corresponding to position 125, may include glycine, alanine, leucine, isoleucine, serine, proline, phenylalanine, tryptophan, methionine, arginine, threonine, cysteine, tyrosine, asparagine, glutamine, lysine, histidine, aspartic acid, and glutamic acid; the amino acid other than ii) glutamine, the amino acid residue corresponding to position 60, may include glycine, alanine, leucine, isoleucine, serine, proline, phenylalanine, tryptophan, methionine, arginine, threonine, cysteine, tyrosine, asparagine, valine, lysine, histidine, aspartic acid, and glutamic acid; the amino acid other than iii) phenylalanine, the amino acid residue corresponding to position 180, may include glycine, alanine, leucine, isoleucine, serine, proline, valine, tryptophan, methionine, arginine, threonine, cysteine, tyrosine, asparagine, glutamine, lysine, histidine, aspartic acid, and glutamic acid; and/or the amino acid other than iv) leucine, the amino acid residue corresponding to position 398, may include glycine, alanine, isoleucine, serine, proline, phenylalanine, valine, tryptophan, methionine, arginine, threonine, cysteine, tyrosine, asparagine, glutamine, lysine, histidine, aspartic acid, and glutamic acid, but are not limited thereto.

More specifically, in the variant of the present disclosure, from the N-terminus of the amino acid sequence of SEQ ID NO: 1, i) valine, the amino acid residue corresponding to position 125, may be substituted with isoleucine; or furthermore, ii) the amino acid residue corresponding to position 60 may be glutamine or arginine, iii) the amino acid residue corresponding to position 180 may be phenylalanine or leucine, and iv) the amino acid residue corresponding to position 398 may be leucine or proline.

Still more specifically, in the variant of the present disclosure, from the N-terminus of the amino acid sequence of SEQ ID NO: 1, i) valine, the amino acid residue corresponding to position 125, may be substituted with isoleucine; or furthermore, ii) glutamine, the amino acid corresponding to position 60, may be substituted with arginine, iii) phenylalanine, the amino acid residue corresponding to position 180, may be substituted with leucine, and/or iv) leucine, the amino acid residue corresponding to position 398, may be substituted with proline.

In an embodiment, the variant of the present disclosure may be a variant consisting of an amino acid sequence having sequence identity of at least 99% with at least any one amino acid sequence selected from SEQ ID NO: 2 or SEQ ID NO: 3.

Alternatively, the variant of the present disclosure may have, contain, consist of, or consist essentially of at least any one amino acid sequence selected from SEQ ID NO: 2 or SEQ ID NO: 3, but is not limited thereto.

In accordance with another aspect of the present disclosure, there is provided a polynucleotide encoding the variant of the present disclosure.

The variant is as described above.

As used herein, the term "polynucleotide" refers to a polymer of nucleotide units linked in a long chain type through covalent linkage, and to a DNA or RNA strand having a predetermined length or longer. More specifically, the term refers to a polynucleotide fragment encoding the variant.

The polynucleotide encoding the variant of the present disclosure may contain a nucleotide sequence encoding the amino acid sequence set forth in SEQ ID NO: 2 or SEQ ID NO: 3. As an example of the present disclosure, the polynucleotide of the present disclosure may have or contain the nucleic acid nucleotide sequence of SEQ ID NO: 4 or SEQ ID NO: 5. In addition, the polynucleotide of the present disclosure may consist of or consist essentially of the nucleic acid nucleotide sequence of SEQ ID NO: 4 or SEQ ID NO: 5.

The polynucleotide of the present disclosure may be variously modified in a coding region thereof within the range in which the amino acid sequence of the variant of the present disclosure is not changed, considering codon degeneracy, or a codon preferred by an organism in which the variant of the present disclosure is to be expressed. Specifically, the polynucleotide of the present disclosure may have or contain a nucleotide sequence having homology or identity of at least 70%, at least 75%, at least 80%, at least 85%, at least 90%, at least 95%, at least 96%, at least 97%, at least 98%, or at least 99%, and less than 100% with the nucleic acid nucleotide sequence of SEQ ID NO: 4 or 5, or may consist of or consist essentially of a nucleotide sequence having homology or identity of at least 70%, at least 75%, at least 80%, at least 85%, at least 90%, at least 95%, at least 96%, at least 97%, at least 98%, or at least 99%, and less than 100% with the nucleic acid nucleotide sequence of SEQ ID NO: 4 or 5, but is not limited thereto. In the sequences having the above homology or identity, a codon encoding the amino acid corresponding to position 125 of SEQ ID NO: 1 may be one of the codons encoding isoleucine.

In addition, the polynucleotide of the present disclosure may include, without limitation, a sequence that can hybridize, under stringent conditions, with a probe that can be prepared from a known gene sequence, for example, a sequence complementary to a part or the entirety of the polynucleotide sequence of the present disclosure. The "stringent conditions" refer to conditions that enable specific hybridization between polynucleotides. Such conditions are specifically described in the literature (see J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York, 9.50-9.51, 11.7-11.8). For example, such conditions may include conditions under which polynucleotides having high homology or identity, such as polynucleotides having at least 70%, at least 75%, at least 80%, at least 85%, at least 90%, at least 95%, at least 96%, at least 97%, at least 98%, or at least 99% homology or identity, hybridize with each other, but under which polynucleotides having lower homology or identity do not hybridize with each other; or washing conditions for typical Southern hybridization, wherein washing is conducted at a salt concentration and a temperature, corresponding to 60°C, 1 ×SSC, 0.1% SDS; specifically 60°C, 0.1×SSC, 0.1% SDS; and more specifically 68°C, 0.1×SSC, 0.1% SDS, once, specifically twice or three times.

The hybridization requires that two nucleic acids have complementary sequences, although mismatches between bases are allowable depending on hybridization stringency. The term "complementary" is used to describe the relationship between nucleotide bases that can hybridize with each another. For example, as for DNA, adenine is complementary to thymine, and cytosine is complementary to guanine. Therefore, the polynucleotide of the present disclosure may also include not only substantially similar nucleic acid sequences but also isolated nucleic acid fragments complementary to the entire sequence.

Specifically, a polynucleotide having homology or identity with the polynucleotide of the present disclosure may be detected using hybridization conditions including a step of hybridization at Tₘ of 55°C and utilizing the above-described conditions. In addition, the Tₘ value may be 60°C, 63°C, or 65°C, but is not limited thereto, and may be appropriately controlled by a person skilled in the art according to the purpose.

The appropriate stringency for hybridizing polynucleotides depends on the length of the polynucleotides and the degree of complementarity, and variables thereof are well known in the art (*e.g*., Sambrook *et al., supra*).

In accordance with still another aspect of the present disclosure, there is provided a vector containing the polynucleotide of the present disclosure. The vector may be an expression vector for expressing the polynucleotide in a host cell, but is not limited thereto.

The polynucleotide is as described above.

The vector of the present disclosure may include a DNA construct containing the nucleotide sequence of the polynucleotide encoding the target polypeptide, operably linked to an appropriate expression control region (or expression control sequence) so as to express the target polypeptide within an appropriate host. The expression control region may include a promoter capable of initiating transcription, any operator sequence for controlling such transcription, a sequence for encoding a suitable mRNA ribosomal binding site, and a sequence for controlling the termination of transcription and translation. The vector, after transformation into an appropriate host, can replicate or function independently of the genome of the host, or may be integrated into the genome itself.

The vector used in the present disclosure is not particularly limited, and any vector known in the art may be used. Examples of the vector commonly used may include naturally occurring or recombinant plasmids, cosmids, viruses, and bacteriophages. For example, pWE15, M13, MBL3, MBL4, IXII, ASHII, APII, t10, t11, Charon4A, Charon21A, and the like may be used as phage vectors or cosmid vectors, and pBR-based, pUC-based, pBluescriptll-based, pGEM-based, pTZ-based, pCL-based, pSK-based, pSKH-based, and pET-based vectors may be used as plasmid vectors. Specifically, pCL, pDC, pDCM2, pSK, pSKH130, pDZ, pACYC177, pACYC184, pECCG117, pUC19, pBR322, pMW118, pCC1BAC, and other vectors may be used.

For example, the polynucleotide encoding the target polypeptide may be inserted into a chromosome through a vector for chromosomal insertion in a cell. The insertion of the polynucleotide into the chromosome may be performed using any method known in the art, for example, homologous recombination, but is not limited thereto. The vector may further include a selection marker for identifying the insertion of the chromosome. The selection marker is for selecting cells transformed with the vector, that is, identifying the insertion of a target nucleic acid molecule, and markers for imparting a selectable phenotype, such as drug resistance, auxotrophy, cytotoxic drug resistance, or expression of surface polypeptides, may be used. In an environment treated with a selective agent, only the cells expressing a selection marker survive or exhibit a different phenotype, and thus the transformed cells can be selected.

As used herein, the term "transformation" means that a vector containing a polynucleotide encoding a target polypeptide is introduced into a host cell or a microorganism to allow the polypeptide encoded by the polynucleotide to be expressed in the host cell. The transformed polynucleotide may include any polynucleotide as long as it can be expressed in a host cell, regardless of whether the polynucleotide is inserted and located in a chromosome or located outside of a chromosome in the host cell. In addition, the polynucleotide includes DNA and/or RNA encoding the target protein. The polynucleotide may be introduced in any form as long as the polynucleotide can be introduced and expressed in the host cell. For example, the polynucleotide may be introduced into a host cell in the form of an expression cassette, which is a gene construct containing all factors required for self-expression. The expression cassette may generally include a promoter operably linked to the polynucleotide, a transcription termination signal, a ribosome binding site, and a translation terminal signal. The expression cassette may be in the form of an expression vector enabling selfreplication. In addition, the polynucleotide may be introduced in its own form into the host cell to be operably linked to a sequence required for expression in the host cell, but is not limited thereto.

As above, the term "operably linked" refers to a functional linkage between the polypeptide sequence and a promoter sequence for initiating and mediating the transcription of the polynucleotide encoding the target protein of the present disclosure.

In accordance with still another aspect of the present disclosure, there is provided a microorganism of the genus *Escherichia,* including a MdtH variant, in which the amino acid corresponding to position 125 in the amino acid sequence of SEQ ID NO: 1 is substituted with another amino acid, or a polynucleotide encoding the variant.

The strain of the present disclosure may include the modified polypeptide of the present disclosure, the polynucleotide encoding the polypeptide, and/or the vector including the polynucleotide of the present disclosure.

The variant, polynucleotide, and vector are as described above.

As used herein, the term "microorganism (or strain)" encompasses all of wild-type microorganisms, or microorganisms with naturally occurring or artificially genetic modifications, and refers to a microorganism in which a particular mechanism is weakened or enhanced due to the insertion of an exogenous gene or the enhancement or inactivation of activity of an endogenous gene, wherein the microorganism may have a genetic modification for production of a target polypeptide, protein, or product.

The strain of the present disclosure may be: a strain containing at least one of the variant of the present disclosure, the polynucleotide of the present disclosure, and the vector including the polynucleotide of the present disclosure; a strain modified to express the variant of the present disclosure or the polynucleotide of the present disclosure; a strain (*e.g*., recombinant strain) expressing the variant of the present disclosure or the polynucleotide of the present disclosure; or a strain (*e.g*., recombinant strain) having the variant activity of the present disclosure, but is not limited thereto.

The strain of the present disclosure may be a strain having an OPS export ability.

The strain of the present disclosure may be a microorganism natively having MdtH or an OPS export ability, or a microorganism obtained by introducing the variant of the present disclosure or the polynucleotide encoding the variant (or the vector including the polynucleotide) into a parent strain without MdtH or an OPS export ability and/or a microorganism imparted with an OPS export ability, but is not limited thereto.

For example, the strain of the present disclosure is a cell or microorganism, which is transformed with a vector containing the polynucleotide of the present disclosure or a polynucleotide encoding the variant of the present disclosure to express the variant of the present disclosure, and for the purpose of the present disclosure, the strain of the present disclosure may include all microorganisms capable of exporting OPS, including the variant of the present disclosure. For example, the strain of the present disclosure may be a recombinant strain having an increased OPS export ability through the expression of the MdtH variant by introducing a polynucleotide encoding the variant of the present disclosure into a naturally occurring wild-type microorganism or a microorganism exporting OPS. The recombinant strain having an increased OPS export ability may be a microorganism in which an OPS export ability is increased compared with a naturally occurring wild-type microorganism or a MdtH-non-modified microorganism (i.e., a microorganism expressing wild-type MdtH (SEQ ID NO: 1) or a microorganism not expressing the modified protein (SEQ ID NO: 2 or 3)), but is not limited thereto. For example, the MdtH-non-modified microorganism, which is an object strain for comparison of the increase in OPS export ability, may be CA07-0012 (KCCM 11121P, European Patent Publication EP 2444481 or U.S. Patent Application Publication No. 2012-0190081), which is a strain having a weakening in endogenous activity of phosphoserine phosphatase (serB), but is not limited thereto.

For example, a recombinant strain with an increased OPS producing ability due to the increased OPS export ability may have an OPS producing ability which is increased by at least about 1%, specifically at least about 1%, at least about 2.5%, at least about 5%, at least about 7.5%, at least about 10%, at least about 12.5%, at least about 15%, at least about 17.5%, at least about 20%, at least about 22.5%, at least about 25%, at least about 27.5%, at least about 30%, at least about 32.5%, at least about 35%, at least about 37.5%, at least about 40%, at least about 42.5%, at least about 45%, at least about 47.5%, at least about 50%, at least about 52.5%, at least about 55%, at least about 57.5%, at least about 60%, at least about 62.5%, at least about 65%, at least about 67.5%, at least about 70%, at least about 72.5%, at least about 75%, at least about 77.5%, at least about 80%, at least about 82.5%, at least about 90%, at least about 92.5%, at least about 95%, at least about 97.5%, at least about 100%, at least about 102.5%, at least about 105%, at least about 107.5%, at least about 110%, at least about 112.5%, at least about 115%, at least about 117.5%, at least about 120%, at least about 122.5%, at least about 125%, at least about 127.5%, at least about 130%, at least about 132.5%, at least about 135%, or at least about 137% (the upper bound is not particularly limited, and the upper bound may be, for example, at most about 300%, at most about 200%, at most about 150%, at most about 100%, at most about 50%, at most about 40%, or at most about 30%) compared with the OPS producing ability of the parent strain before modification or a non-modified microorganism, but the OPS producing ability is not limited thereto as long as it has an increase of a positive value compared with the producing ability of the parent strain before modification or a non-modified microorganism. In another example, a recombinant strain with an increased OPS producing ability due to the increased OPS export ability may have an OPS producing ability which is increased by at least about 1.01 times, at least about 1.025 times, at least about 1.05 times, at least about 1.075 times, at least about 1.10 times, at least about 1.125 times, at least about 1.15 times, at least about 1.175 times, at least about 1.20 times, at least about 1.225 times, at least about 1.25 times, at least about 1.275 times, at least about 1.30 times, at least about 1.325 times, at least about 1.35 times, at least about 1.375 times, at least about 1.50 times, at least about 1.525 times, at least about 1.55 times, at least about 1.60 times, at least about 1.625 times, at least about 1.65 times, at least about 1.675 times, at least about 1.70 times, at least about 1.725 times, at least about 1.75 times, at least about1.775 times, at least about 1.8 times, at least about 1.825 times, at least about 1.85 times, at least about 1.875 times, at least about 1.90 times, at least about 1.925 times, at least about times, 1.95 times, at least about 1.975 times, at least about 2.0 times, at least about 2.025 times, at least about 2.05 times, at least about 2.075 times, at least about 2.10 times, at least about 2.125 times, at least about 2.15 times, at least about 2.175 times, at least about 2.20 times, at least about 2.225 times, at least about 2.25 times, at least about 2.275 times, at least about 2.30 times, at least about 2.325 times, at least about 2.35 times, or at least about 2.37 times (the upper bound is not particularly limited, and the upper bound may be, for example, at most about 10 times, at most about 5 times, at most about 3 times, or at most about 2 times) compared with the OPS producing ability of the parent strain before modification or a non-modified microorganism, but is not limited thereto. As used herein, the term "about" refers to a range including ±0.5, ±0.4, ±0.3, ±0.2, ±0.1, and the like, and thus includes all of the values in the range equivalent or similar to those stated after this term, but is not limited thereto.

As used herein, the term "non-modified microorganism" may refer to a wild-type strain or a naturally occurring strain as it is, or a strain before the change of a trait thereof due to a genetic modification caused by a natural or artificial cause, not excluding strains containing naturally occurring mutations in microorganisms. For example, the non-modified microorganism may refer to a strain into which the MdtH variant described herein is not introduced or before the MdtH variant is introduced. The "non-modified microorganism" may be used interchangeably with "strain before modification", "microorganism before modification", "nonmutated strain", "non-modified strain", "non-modified microorganism", or "reference microorganism".

As still another example of the present disclosure, the microorganism of the present disclosure is not particularly limited to the kind thereof as long as it can produce OPS, and the microorganism of the present disclosure may be both a prokaryotic cell or a eukaryotic cell, and specifically a prokaryotic cell. Examples of the prokaryotic cells may include microorganism strains pertaining to the genus *Escherichia,* the genus *Erwinia,* the genus *Seratia,* the genus *Providencia,* the genus *Corynebacterium,* and the genus *Brevibacterium,* and the prokaryotic cell may be specifically a microorganism of the genus *Escherichia,* and more specifically *Escherichia coli,* but is not limited thereto.

In particular, the microorganism of the genus *Escherichia* of the present disclosure can produce OPS and L-serine through SerA, SerC, and SerB, which are enzymes of the biosynthesis pathway of L-serine (Ahmed Zahoor, Computational and structural biotechnology journal, Vol. 3, 2012 October; Wendisch V. F. et al., Curr Opin Microbiol. 2006 Jun;9(3):268-74; and Peters-Wendisch P. et al., Appl Environ Microbiol. 2005 Nov;71(11):7139-44). SerB, which is a phosphoserine phosphatase, has the activity to convert OPS to L-serine, so that a microorganism modified to have weakened activity of SerB has a property of accumulating OPS and thus can be advantageously used in the production of OPS. For example, the microorganism of the present disclosure may be a recombinant microorganism having further weakened activity of SerB compared with the endogenous activity thereof, in addition to the introduction of the variant of the present disclosure.

SerB of the present disclosure may be a protein having or containing the amino acid sequence set forth in NCBI Accession No. AAC77341.1, or a protein consisting of or consisting essentially of the amino acid sequence set forth in AAC77341.1, but is not limited thereto. In addition, SerB of the present disclosure may have or contain an amino acid sequence having homology or identity of at least 70%, 80%, 90%, 95%, or 99% with the amino acid sequence set forth in AAC77341.1 as long as SerB of the present disclosure exhibits the conversion activity of OPS into L-serine. Additionally, SerB of the present disclosure may consist or essentially consist of an amino acid sequence having homology or identity of at least 70%, 80%, 90%, 95%, or 99% with the amino acid sequence set forth in AAC77341.1, but is not limited thereto.

Furthermore, the polynucleotide encoding SerB of the present disclosure may have or contain the amino acid sequence set forth in NCBI NP_415583.4. The polynucleotide encoding SerB of the present disclosure may have or contain an amino acid sequence having homology or identity of at least 70%, 80%, 90%, 95%, or 99%, and less than 100% with the nucleotide sequence of NP_415583.4. Additionally, the polynucleotide encoding SerB of the present disclosure may consist of or consist essentially of an amino acid sequence having homology or identity of at least 70%, 80%, 90%, 95%, or 99%, and less than 100% with the nucleotide sequence of NP_415583.4, but is not limited thereto.

As used herein, the term "enhancement" in activity of a polypeptide refers to an increase in activity of the polypeptide compared with the endogenous activity thereof. The enhancement may be used interchangeably with activation, up-regulation, overexpression, increase, or the like. The activation, enhancement, up-regulation, overexpression, and increase may include all of exhibiting an activity that has not been natively possessed or exhibiting an activity improved compared with the endogenous activity or activity before modification. The "endogenous activity" refers to the activity of a particular polypeptide, natively possessed by a parental strain before the change of a trait thereof, or a non-modified microorganism, when the trait is changed due to a genetic modification caused by a natural or artificial cause. This term may be used interchangeably with "activity before modification". The "enhancement", "up-regulation", "overexpression", or "increase" in activity of a polypeptide compared with the endogenous activity thereof means an improvement in activity and/or concentration (expression level) of a particular polypeptide natively possessed by a parent strain before the change of a trait thereof or a non-modified microorganism.

The enhancement can be achieved by the introduction of an exogenous polypeptide or through the enhancement of the inherent activity of the polypeptide and/or the concentration (expression level) thereof. The enhancement of the activity of the polypeptide can be identified by an increase in activity degree or expression level of the corresponding polypeptide or an increase in amount of a product produced from the corresponding polypeptide.

The enhancement in activity of the polypeptide may be achieved by applying various methods well known in the art, and is not limited as long as the method can enhance the activity of a target polypeptide compared with the microorganism before modification. Specifically, gene engineering and/or protein engineering well known to a person skilled in the art, which is a routine method of molecular biology, may be used, but is not limited thereto (*e.g*., Sitnicka et al. Functional Analysis of Genes. Advances in Cell Biology 2010, Vol. 2. 1-16, Sambrook et al. Molecular Cloning 2012, *etc*.).

Specifically, the enhancement in activity of the polypeptide of the present disclosure may be:
1) an increase in intracellular copy number of the polynucleotide encoding the polypeptide;
2) a modification of a gene expression control region on the chromosome encoding the polypeptide;
3) a modification of a nucleotide sequence encoding an initiation codon or 5'-UTR region of the gene transcript encoding the polypeptide;
4) a modification of the amino acid sequence of the polypeptide so as to enhance activity of the polypeptide;
5) a modification of the polynucleotide sequence encoding the polypeptide so as to enhance activity of the polypeptide (*e.g*., a modification of the polynucleotide sequence of the polypeptide gene so as to encode a polypeptide modified to enhance activity of the polypeptide);
6) an introduction of an exogenous polypeptide exhibiting activity of the polypeptide or an exogenous polypeptide encoding the same;
7) a codon optimization of the polynucleotide encoding the polypeptide;
8) a modification or chemical modification of an exposure site selected by analysis of a tertiary structure of the polypeptide; or
9) a combination of two or more selected from items 1) to 8), but is not particularly limited thereto.

More specifically, these are described as follows.

The increase in intracellular copy number of the polynucleotide encoding the polypeptide in item 1) may be achieved by an introduction, into a host cell, of a vector to which the polynucleotide encoding the corresponding polypeptide is operably linked and which can replicate and function independently of a host. Alternatively, the increase in item 1) may be achieved by an introduction of one copy or two or more copies of the polynucleotide encoding the corresponding polypeptide into a chromosome in a host cell. The introduction into the chromosome may be performed by an introduction, into a host cell, of a vector capable of inserting the polynucleotide into the chromosome in the host cell, but is not limited thereto. The vector is as described above.

The modification of a gene expression control region (or expression control sequence) on the chromosome encoding the polypeptide in item 2) may be, for example, a modification occurring in the sequence through a deletion, an insertion, a non-conservative or conservative substitution, or a combination thereof, or a replacement with or insertion of a sequence having stronger activity, so as to further enhance the activity of the expression control region. The expression control region may include, but is not particularly limited to, a promoter, an operator sequence, a sequence encoding a ribosome binding site, a sequence controlling the termination of transcription and translation, and the like. For example, it may be a replacement of the native promoter with a stronger promoter, but is not limited thereto.

Known examples of the stronger promoter may be CJ1 to CJ7 promoters (U.S. Patent US 7662943 B2), lac promoter, trp promoter, trc promoter, tac promoter, lamda phage PR promoter, PL promoter, tet promoter, gapA promoter, SPL7 promoter, SPL13 (sm3) promoter (U.S. Patent US 10584338 B2), O2 promoter (U.S. Patent US 10273491 B2), tkt promoter, yccA promoter, and the like, but are not limited thereto.

The modification of a nucleotide sequence encoding an initiation codon or 5'-UTR region of the gene transcript encoding the polypeptide in item 3) may be, for example, a substitution with a nucleotide sequence encoding, rather than an endogenous initiation codon, another initiation codon having a higher expression level of the polypeptide, but is not limited thereto.

The modification of the amino acid sequence or the polynucleotide sequence in items 4) and 5) may be a modification occurring in the sequence through a deletion, an insertion, a non-conservative or conservative substitution, or a combination thereof in the amino acid sequence of the polypeptide or the polynucleotide sequence encoding the polypeptide, or a replacement with an amino acid sequence or polynucleotide sequence modified to have stronger activity or an amino acid sequence or polynucleotide sequence modified to have increased activity, so as to enhance activity of the polypeptide, but is not limited thereto. Specifically, the replacement may be performed by inserting the polynucleotide into the chromosome by homologous recombination, but is not limited thereto. The vector used herein may further include a selection marker for identifying the insertion of the chromosome. The selection marker is as described above.

The introduction of an exogenous polypeptide exhibiting activity of the polypeptide in item 6) may be an introduction, into a host cell, of an exogenous polynucleotide encoding a polypeptide exhibiting the same/similar activity with regard to the polypeptide. The exogenous polynucleotide is not limited to the origin or sequence thereof as long as the exogenous polynucleotide exhibits the same/similar activity with regard to the polynucleotide. The introduction may be performed by any known transformation method that is appropriately selected by a person skilled in the art, and through the expression of the introduced polynucleotide in the host cell, the polypeptide can be produced, and the activity thereof can be enhanced.

The codon optimization of the polynucleotide encoding the polypeptide in item 7) may be the codon optimization of an endogenous polynucleotide so as to increase transcription or translation thereof in a host cell, or the codon optimization of an exogenous polynucleotide so as to allow optimized transcription or translation thereof in a host cell.

The modification or chemical modification of an exposure site selected by analysis of a tertiary structure of the polypeptide in item 8) may be, for example, that the sequence information of a polypeptide to be analyzed is compared with a database storing sequence information of base proteins to determine template protein candidates according to the degree of sequence similarity, followed by identification of structures on the basis of the candidates, to thereby select an exposure site to be modified or chemically modified, and the exposure site is then modified or chemically modified.

Such an enhancement of the activity of the polypeptide may mean that the activity, concentration, or expression level of the corresponding polypeptide is increased relative to the activity or concentration of the polypeptide expressed in a wild-type microorganism strain or a microorganism before modification, or the amount of a product produced from the corresponding polypeptide is increased, but is not limited thereto.

As used herein, the term "weakening" of activity of the polypeptide has a concept encompassing all of the decrease of activity or the absence of activity compared with the endogenous activity. The weakening may be used interchangeably with inactivation, deficiency, down-regulation, decrease, reduction, attenuation, or the like.

The weakening may also include: a case where the activity of the polypeptide itself is decreased or eliminated compared with the activity of the polypeptide natively possessed by the microorganism due to a modification or the like of the polynucleotide encoding the polypeptide; a case where the activity and/or concentration (expression level) of the entire polypeptide in the cell is lower compared with the naturally occurring strain due to the inhibition of the expression of a gene of a polynucleotide encoding the polypeptide or the inhibition of the translation into the polypeptide; a case where the expression of the polynucleotide is not made; and/or a case where the polypeptide has no activity in spite of the expression of the polynucleotide. The "endogenous activity" refers to the activity of a particular polypeptide natively possessed by a parent strain before the change of a trait thereof or a wild-type or non-modified microorganism when the trait is changed due to a genetic modification caused by a native or artificial cause. This term may be used interchangeably with the "activity before modification". The "inactivation, deficiency, decrease, down-regulation, reduction, or attenuation" of the activity of a polypeptide compared with the endogenous activity thereof means that the activity of the polypeptide is lowered compared with the activity of a particular polypeptide natively possessed by a parent strain before the change of a trait thereof or a non-modified microorganism.

The weakening of the activity of the polypeptide may be achieved by way of any method known in the art, but is not limited thereto, and may be achieved by applying various methods well known in the art (*e.g*., Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of the polypeptide of the present disclosure may be:
1) a deletion of a part or the entirety of the gene encoding the polypeptide;
2) a modification of an expression control region (or expression control sequence) so as to decrease the expression of the gene encoding the polypeptide;
3) a modification of the amino acid sequence constituting the polypeptide so as to eliminate or weaken the activity of the polypeptide (*e.g*., deletion/substitution/addition of at least one amino acid in the amino acid sequence);
4) a modification of the gene sequence encoding the polynucleotide so as to eliminate or weaken the activity of the polypeptide (*e.g*., deletion/substitution/addition of at least one nucleic acid nucleotide in the nucleic acid nucleotide sequence of the polypeptide gene so as to encode the polypeptide modified to eliminate or weaken the activity of the polypeptide);
5) a modification of a nucleotide sequence encoding the initiation codon or 5'-UTR region of the gene transcript encoding the polypeptide;
6) an introduction of an antisense oligonucleotide (*e.g*., antisense RNA) complementarily binding to the gene transcript encoding the polypeptide;
7) an addition of a sequence complementary to the Shine-Dalgarno sequence of the gene encoding the polypeptide to the upstream of the Shine-Dalgarno sequence so as to form a secondary structure that makes the attachment of ribosomes impossible;
8) an addition of a promoter, which is to be reverse-transcribed, to the 3' end of the open reading frame (ORF) of the gene sequence encoding the polypeptide (reverse transcription engineering, RTE); or
9) a combination of two or more selected from items 1) to 8), but is not particularly limited thereto.

For example, these are described as follows.

The deletion of a part or the entirety of the gene encoding the polypeptide in item 1) may be an elimination of the entirety of the polynucleotide encoding an endogenous target protein in the chromosome, a replacement with a polynucleotide with a deletion of some nucleotides, or a replacement with a marker gene.

The modification of an expression control region (or expression control sequence) in item 2) may be a modification occurring in the expression control region (or expression control sequence) through a deletion, an insertion, a non-conservative or conservative substitution, or a combination thereof, or a replacement with a sequence having weaker activity. The expression control region includes a promoter, an operator sequence, a sequence for encoding a ribosomal binding site, and a sequence for controlling the termination of transcription and translation, but is not limited thereto.

The modification of a nucleotide sequence encoding the initiation codon or 5'-UTR region of the gene transcript encoding the polypeptide in item 5) may be, for example, a substitution with a nucleotide sequence encoding, rather than the endogenous initiation codon, another initiation codon having a lower expression level of the polypeptide, but is not limited thereto.

The modification of the amino acid sequence or the polynucleotide sequence in items 3) and 4) may be a modification occurring in the sequence through a deletion, an insertion, a non-conservative or conservative substitution, or a combination thereof in the amino acid sequence of the polypeptide or the polynucleotide sequence encoding the polypeptide, or a replacement with an amino acid sequence or polynucleotide sequence modified to have weaker activity or an amino acid sequence or polynucleotide sequence modified to have no activity, so as to weaken the activity of the polypeptide, but is not limited thereto. For example, the expression of the gene may be inhibited or weakened by introducing a modification into the polynucleotide sequence to form a termination codon.

The introduction of an antisense oligonucleotide (*e.g*., antisense RNA) complementarily binding to the gene transcript encoding the polypeptide in item 6) may be referred to, for example, in the literature reference Weintraub, H. et al., Antisense-RNA as a molecular tool for genetic analysis, Reviews - Trends in Genetics, Vol. 1(1) 1986.

The addition of a sequence complementary to the Shine-Dalgarno sequence of the gene encoding the polypeptide to the upstream of the Shine-Dalgarno sequence so as to form a secondary structure that makes the attachment of ribosomes impossible in item 7) may be making mRNA translation impossible or reducing the rate thereof.

The addition of a promoter, which is to be reverse-transcribed, to the 3' end of the open reading frame (ORF) of the gene sequence encoding the polypeptide (reverse transcription engineering, RTE) in item 8) may be the preparation of an antisense nucleotide complementary to the transcript of the gene encoding the polypeptide to thereby weaken the activity of the polypeptide.

The modification of a part or the entirety of the polynucleotide in the microorganism of the present disclosure may be induced by: (a) genome editing adopting homologous recombination or engineered nuclease (*e.g*., CRISPR-Cas9) using a vector for chromosomal insertion in a microorganism or and/or (b) the treatment with light, such as ultraviolet light and radiation, and/or chemicals, but is not limited thereto. The method of modifying a part or the entirety of the gene may include a method by DNA recombinant technology. For example, a nucleotide sequence or vector containing a nucleotide sequence homologous to a target gene may be injected into the microorganism to bring about homologous recombination, resulting in a deletion in a part or the entirety of the gene. The nucleotide sequence or vector to be injected may include a dominant selection marker, but is not limited thereto.

In addition, the microorganism may be a microorganism further having a decrease in ability to introduce OPS into cells or degrade OPS.

Regarding the OPS producing microorganisms as above, the disclosure of European Patent Publication EP 2444481 or U.S. Patent Application Publication No. 2012-0190081 may be used as reference materials of the present disclosure, but is not limited thereto.

In the microorganism of the present disclosure, the variant, polynucleotide, OPS, and the like are as described in the other aspects.

In accordance with still another aspect of the present disclosure, there is provided a method for producing O-phosphoserine, the method including culturing in a medium a microorganism containing a MdtH variant, in which the amino acid corresponding to position 125 in the amino acid sequence of SEQ ID NO: 1 is substituted with another amino acid, or a polynucleotide encoding the variant.

The OPS production method of the present disclosure may include culturing in a medium a microorganism containing the variant of the present disclosure, the polynucleotide of the present disclosure, or the vector of the present disclosure.

As used herein, the term "culture" refers to growing the microorganism of the present disclosure in appropriately adjusted environment conditions. The culture procedure of the present disclosure may be performed according to appropriate media or culture conditions known in the art. This culture procedure may be easily adjusted and employed depending on the selected strain by a person skilled in the art. Specifically, the culture may be batch culture, continuous culture, and/or fed-batch culture, but is not limited thereto.

As used herein, the "medium" refers to a mixture containing as main ingredients nutrient substances required for culturing the microorganism of the present disclosure, wherein the medium supplies nutrient substances, growth factors, and the like, including water that is essential for survival and growth. Specifically, as for the medium and other culture conditions used for culturing the microorganism of the present disclosure, any medium that is used to culture typical microorganisms may be used without particular limitation. However, the microorganisms of the present disclosure may be cultured under aerobic conditions in a conventional medium containing appropriate carbon sources, nitrogen sources, phosphorus sources, inorganic compounds, amino acids, and/or vitamins, while the temperature, pH, and the like are adjusted.

Examples of the carbon source to be contained in the medium may include: saccharides and carbohydrates, such as glucose, sucrose, lactose, fructose, maltose, starch, and cellulose; oils and fats, such as soybean oil, sunflower oil, castor oil, and coconut oil; fatty acids, such as palmitic acid, stearic acid, and linoleic acid; alcohols, such as glycerol and ethanol; and organic acids such as acetic acid. These materials may be used individually or in a mixture, but are not limited thereto.

Examples of the nitrogen source to be contained in the medium may include: organic nitrogen sources, such as peptone, yeast extract, gravy, malt extract, corn steep liquor, and bean flour; and inorganic nitrogen sources, such as urea, ammonium sulfate, ammonium chloride, ammonium phosphate, ammonium carbonate, and ammonium nitrate. These nitrogen sources may be used alone or in combination, but are not limited thereto

As phosphorous sources to be contained in the medium, potassium dihydrogen phosphate, dipotassium hydrogen phosphate, and corresponding sodium-containing salts may be contained, but are not limited thereto.

The medium may contain metal salts, such as magnesium sulfate and iron sulfate, and additionally, may contain amino acids, vitamins, and appropriate precursors. These media or precursors may be added to cultures in a batch or continuous manner, but are not limited thereto.

The pH of cultures may be adjusted by adding a compound, such as ammonium hydroxide, potassium hydroxide, ammonia, phosphoric acid, and sulfuric acid, to the cultures in an appropriate manner during the culture. In addition, a antifoaming agent, such as a fatty acid polyglycol ester, may be added to suppress foam formation. In addition, oxygen or oxygen-containing gas may be injected into the medium to maintain the aerobic state of the medium, or no gas, or nitrogen, hydrogen, or carbon dioxide gas may be injected to maintain the anaerobic or non-aerobic state of the medium, but is not limited thereto.

In the culture of the present invention, the culture temperature may be maintained at 20°C to 45°C, and specifically 30°C to 35°C, and the culture period may continue until a desired production of a useful substance can be obtained, and may be specifically 10 to 100 hours. However, the culture temperature and the culture period are not limited thereto.

OPS produced by the culture of the present invention may be released into the medium.

The OPS production method of the present invention may further include preparing the microorganism of the present disclosure, preparing a medium for culturing the microorganism, or a combination thereof (regardless of the order, in any order), for example, before or after the culturing step.

The OPS production method of the present disclosure may further include recovering OPS from a medium resulting from the culturing (a medium in which the culturing has been performed) or the microorganism of the present disclosure. The method may further include the recovering step after the culturing step.

The method of recovering OPS may be collecting desired OPS by using an appropriate method known in the art according to the method for culturing a microorganism of the present disclosure, for example, a batch, continuous, or fed-batch culture method. For example, centrifugation, filtration, treatment with a crystallized protein precipitating agent (salting-out), extraction, ultrasonic disruption, ultrafiltration, dialysis, various types of chromatography, such as molecular sieve chromatography (gel filtration), adsorption chromatography, ion exchange chromatography, and affinity chromatography, HPLC, and a combination of these methods may be used, and desired OPS may be recovered from the medium or the microorganism by using an appropriate method known in the art.

The OPS production method of the present disclosure may further include a purifying step. The purifying may be performed by using an appropriate method known in the art. In an example, when the OPS production method of the present disclosure includes both the recovering step and the purifying step, the recovering step and the purifying step may be performed continuously or discontinuously regardless of the order, or may be performed simultaneously or integrated into one step, but is not limited thereto.

In the method of the present disclosure, the variant, polynucleotide, vector, strain, and the like are as described in the other aspects.

In accordance with still another aspect of the present disclosure, there is provided a method for producing cysteine or a derivative thereof.

Specifically, the method may include: a) culturing in a medium a microorganism comprising a MdtH variant, in which the amino acid corresponding to position 125 in the amino acid sequence of SEQ ID NO: 1 is substituted with another amino acid, or a polynucleotide encoding the variant, to thereby produce O-phosphoserine or a medium containing the O-phosphoserine; and b) reacting a sulfide with O-phosphoserine sulfhydrylase (OPSS) or a microorganism expressing OPSS and O-phosphoserine or the medium containing O-phosphoserine produced in step a).

As used herein, the term "derivative" refers to similar compound obtained by chemically modifying a portion of a certain compound, and usually, the term refers to a compound in which a hydrogen atom or a particular atom group is substituted with another hydrogen atom or atom group.

As used herein, the term "derivative of cysteine" refers to a compound in which a hydrogen atom or a particular atom group in cysteine is substituted with another atom or atom group. For example, the cysteine derivative may have a form in which the nitrogen atom of the amine group (-NH₂) or the sulfur atom of the thiol group (-SH) in cysteine has another atom or atom group attached thereto. Examples of the cysteine derivative include *N*-acetylcysteine (NAC), *S-*carboxymethylcysteine (SCMC), Boc-Cys(Me)-OH, (*R*)-*S*-(2-amino-2-carboxyethyl)-L-homocysteine, (*R*)-2-amino-3-sulfopropionic acid, D-2-amino-4-(ethylthio)butyric acid, 3-sulfino-L-alanine, Fmoc-Cys(Boc-methyl)-OH, seleno-L-cysteine, *S*-(2-thiazolyl)-L-cysteine, *S*-(2-thienyl)-L-cysteine, and *S*-(4-tolyl)-L-cysteine, but are not limited thereto.

As long as cysteine is produced according to the method of the present disclosure, the conversion into various cysteine derivatives can be easily achieved by way of methods well known in the art.

Specifically, the cysteine derivative production method may further include converting the cysteine produced in step b) into a cysteine derivative. For example, cysteine may react with an acetylation agent to synthesize *N-*acetylcysteine (NAC), or cysteine may react with a haloacetic acid in a basic condition to synthesize S-carboxymethylcysteine (SCMC), but is not limited thereto.

These cysteine derivatives are used mainly as pharmaceutical raw materials for antitussive agents, cough-relieving agents, and therapeutic agents for bronchitis, bronchial asthma, laryngopharyngitis, and the like.

As used herein, the term "*O*-phosphoserine sulfhydrylase (OPSS)" refers to an enzyme that catalyzes a reaction in which a thiol (SH) group is provided to OPS to convert OPS into cysteine. The enzyme may be one that was first found in *Aeropymm pernix, Mycobacterium tuberculosis, Mycobacterium megmatics, Trichomonas vaginalis* (Mino, K. and Ishikawa, K., FEBSletters, 551:133-138, 2003; and Bums, K. E. et al, J. Am. Chem. Soc, 127:11602-11603, 2005). In addition, the OPSS includes not only a wild-type OPSS protein, but also a variant protein that contains a deletion, a substitution, or an addition with respect to a part of the polynucleotide sequence encoding the OPSS and exhibits the activity that is equivalent to or higher than the biological activity of the wild-type OPSS protein, and may encompass all of the OPSS proteins and variant proteins thereof, which are disclosed in European Patent Publication EP 2444481 and U.S. Patent Application Publication US 9127324.

The sulfide may be any sulfide that is provided not only in a solid form commonly used in the art, but also in a liquid or gas form due to a difference in pH, pressure, or solubility and thus can be converted to a thiol (SH) group in the form of, for example, sulfide (S²⁻) or thiosulfate (S₂O₃²⁻). Specifically, Na₂S, NaSH, H₂S, (NH₄)₂S, NaSH, and Na₂S₂O₃ that provide a thiol group to OPS may be used, but are not limited thereto. In the reaction, a single thiol group is provided to a single OPS reactive group to produce a single cysteine or cysteine derivative. In the reaction, the amount of the sulfide added may be 0.1 to 3 times, specifically 1 to 2 times the molar concentration of OPS, but is not limited thereto.

In addition, the present disclosure may further include recovering cysteine produced through the reaction step. The desired cysteine can be collected by separation and purification from the reaction solution through the use of an appropriate reaction known in the art.

In accordance with still another aspect of the present disclosure, there is provided a composition for producing OPS, the composition including: a microorganism containing a MdtH variant in which the amino acid corresponding to position 125 in the amino acid sequence of SEQ ID NO: 1 is substituted with another amino acid, or a polynucleotide encoding the variant; a medium for culturing the microorganism; or a combination of two or more thereof.

The composition of the present disclosure may further contain any appropriate excipient that is usually used in the composition for producing OPS, and examples of the excipient may be a preserving agent, a wetting agent, a dispersing agent, a suspending agent, a buffer, a stabilizing agent, or an isotonic agent, but are not limited thereto.

In the composition of the present disclosure, the variant, polynucleotide, vector, strain, medium, OPS, and the like are as described in the other aspects.

In accordance with still another aspect of the present disclosure, there is provided use of the modified polypeptide exhibiting an OPS export activity of the present disclosure for producing OPS, cysteine, or a derivative of cysteine.

In accordance with still another aspect of the present disclosure, there is provided use of the polypeptide having the modified polypeptide exhibiting an OPS export activity of the present disclosure for exporting OPS from a microorganism.

In the uses of the present disclosure, the variant, polynucleotide, vector, strain, medium, OPS, and the like are as described in the other aspects.

### [Mode for Carrying Out the Invention]

Hereinafter, the present disclosure will be described in detail with reference to examples. However, these examples are intended to illustrate the present disclosure by way of example and the scope of the present disclosure is not limited to these examples.

### Example 1: mdtH library construction and screening

To select a MdtH variant with an increased OPS export activity, an mdtH gene variant plasmid library was constructed. The specific procedure is as follows.

Any mutagenesis PCR was performed using a primer pair of SEQ DI NO: 9 and SEQ ID NO: 10 (primers 3 and 8) while the genomic DNA of *E. coli* K12 W3110 (Genbank:NC_007779.1) was used as a template (Takara Diversify PCR random mutagenesis kit, Cat. No. 630703).

PCR was performed with denaturation at 94°C for 5 minutes, 20 cycles of denaturation at 94°C for 30 seconds, annealing at 55°C for 30 seconds, and extending at 72°C for 1 minute, and then extending at 72°C for 5 minutes.

In order to introduce the mutant gene fragments thus prepared through such a procedure into the pCL1920 vector (GenBank No. AB236930) having the trc promoter, pCL_Ptrc was first prepared. To secure the trc promoter fragment, PCR was performed using a primer pair of SEQ ID NO: 7 and SEQ ID NO: 8 (primers 1 and 2). PCR was performed with denaturation at 94°C for 5 minutes, 30 cycles of denaturation at 94°C for 30 seconds, annealing at 55°C for 30 seconds, and extending at 72°C for 1 minute, and then extending at 72°C for 5 minutes.

The primer sequences used herein are shown in Table 1 below.

**TABLE 1**

| Sequence number | Sequence name | Sequence |
|---|---|---|
| 7 | primer 1 | CACAGGAAAGATatcatgTCCCGCGTGTCGCAGGC |
| 8 | primer 2 | TTGCATGCCTGCAtcaGGCGTCGCGTTC |
| 9 | primer 3 | CACAGGAAAGATatcatgTCCCGCGTGTCGCAGGC |
| 10 | primer 8 | CTTGCATGCCTGCAGGGCAGTAAGGGCAGTGATC |

The trc promoter fragment was cloned into the pCL1920 vector, digested with EcoRI and Sall, by using an in-fusion cloning kit (Clontech Laboratories, Inc.), and pCL_Ptrc was secured. The secured vector pCL_Ptrc was digested with Pstl and EcoRV, and then the mutant gene fragments obtained through PCR were cloned using an infusion cloning kit. Cloning was performed at 50°C for 60 minutes, and through the cloning, the pCL_Ptrc-mdtH gene variant plasmid library was constructed.

The constructed pCL_Ptrc-mdtH gene mutant plasmid library was transformed into CA07-0012 (KCCM 11121P, European Patent Publication EP 2444481 or U.S. Patent Application Publication No. 2012-0190081) by electroporation. Of these, two kinds of strains containing variants were selected, and plasmids were obtained therefrom, and nucleotide sequences thereof were analyzed through sequencing.

As a result of nucleotide sequence analysis, the two selected variants were identified to be a variant in which the 125th amino acid residue valine was substituted with isoleucine in the amino acid sequence of wild-type MdtH [mdtH(V1251)]; and a variant in which the 60th amino acid residue glutamine was substituted with arginine, the 125th amino acid residue valine was substituted with isoleucine, the 180th amino acid residue phenylalanine was substituted with leucine, and the 398th amino acid residue leucine was substituted with proline [mdtH(Q60R, V125I, F180L, L398P)]. CA07-0012/pCL_Ptrc-mdtH(V125I), which was the strain transformed with the variant mdtH(V1251), was named *E. coli* CA07-0379, and CA07-0012/mdtH(Q60R, V125I, F180L, L398P), which was the strain transformed with the variant mdtH(Q60R, V125I, F180L, L398P), was named *E*. *coli* CA07-0380.

### Example 2: Evaluation of OPS producing ability of strains introduced with MdtH variants

The OPS producing ability of strains introduced with MdtH variants was evaluated using the following medium (Table 2).

**TABLE 2**

| Medium ingredients | Amount of preparation |
|---|---|
| Glucose | 40 g |
| KH₂PO₄(KP1) | 6 g |
| (NH₄)₂SO₄ | 17 g |
| MgSO₄·7H₂O | 1 g |
| MnSO₄·4H₂O | 5 mg |
| FeSO₄·7H₂O | 10 mg |
| L-Glycine | 2.5 g/L |
| Yeast extract | 3 g/L |
| CaCO₃ | 30 g/L |
| pH | 6.8 |

Specifically, as for culturing, each strain was plated on an LB solid medium, followed by culture overnight at 33°C in an incubator. The strain cultured on the LB solid medium overnight was seeded in 25 mL of the titer medium of Table 2, and cultured at a temperature of 33°C in an incubator at 200 rpm for 48 hours. The OPS producing ability was evaluated, and the results are shown in Table 3.

**TABLE 3**

| Strain | OPS concentration (g/L) |
|---|---|
| CA07-0012/pCL_Ptrc-mdtH | 1.9 |
| CA07-0012/pCL_Ptrc-mdtH(V1251) | 3.4 |
| CA07-0012/pCL_Ptrc-mdtH(Q60R, V125I, F180L, L398P) | 4.5 |

As a result, CA07-0012/pCL_Ptrc-mdtH(V125I), which is the strain introduced with mdtH (V125I), showed about 180% production ability compared with CA07-0012/pCL_Ptrc-mdtH, which is the strain introduced with wild-type mdtH. In addition, CA07-0012/pCL_Ptrc-mdtH(Q60R, V125I, F180L, L398P), which is the strain introduced with mdtH(Q60R, V125I, F180L, L398P), showed about 237% production ability compared with the strain CA07-0012/pCL_Ptrc-mdtH.

CA07-0012/pCL_Ptrc-mdtH(V125I) was named CA07-0379, and CA07-0012/pCL_Ptrc-mdtH(Q60R, V125I, F180L, L398P) was named CA07-0380.

From the above description, a person skilled in the art to which the present disclosure pertains will be able to understand that the present disclosure may be embodied in other specific forms without departing from the technical spirit or essential characteristics thereof. Therefore, the embodiments described above should be construed as being exemplified and not limiting the present disclosure. The scope of the present disclosure should be understood as including all changes or modifications derived from the definitions and scopes of the claims and their equivalents.

## Claims

1. A MdtH variant, in which the amino acid corresponding to position 125 in the amino acid sequence of SEQ ID NO: 1 is substituted with another amino acid.

2. The MdtH variant of claim 1, wherein the other amino acid is isoleucine.

3. The MdtH variant of claim 2, wherein the amino acid corresponding to position 125 is valine.

4. The MdtH variant of claim 1, wherein the variant has an O-phosphoserine export activity.

5. The MdtH variant of claim 1, wherein the amino acid corresponding to position 60 in the amino acid sequence of SEQ ID NO: 1 is glutamine or arginine.

6. The MdtH variant of claim 1, wherein the amino acid corresponding to position 180 in the amino acid sequence of SEQ ID NO: 1 is phenylalanine or leucine.

7. The MdtH variant of claim 1, wherein the amino acid corresponding to position 398 in the amino acid sequence of SEQ ID NO: 1 is leucine or proline.

8. The MdtH variant of claim 1, wherein the variant has sequence identity of no less than 80% and less than 100% with the amino acid sequence of SEQ ID NO: 1.

9. The MdtH variant of claim 1, wherein the variant is composed of a polypeptide set forth in the amino acid sequence of SEQ ID NO: 2 or SEQ ID NO: 3.

10. A polynucleotide encoding the variant of claim 1.

11. A recombinant microorganism of the genus *Escherichia,* comprising a MdtH variant, in which the amino acid corresponding to position 125 in the amino acid sequence of SEQ ID NO: 1 is substituted with another amino acid, or a polynucleotide encoding the variant.

12. The recombinant microorganism of claim 11, wherein the recombinant microorganism further has a phosphoserine phosphatase (SerB) activity weakened compared with the endogenous activity thereof.

13. A method for producing O-phosphoserine, the method comprising culturing in a medium a microorganism comprising a MdtH variant, in which the amino acid corresponding to position 125 in the amino acid sequence of SEQ ID NO: 1 is substituted with another amino acid, or a polynucleotide encoding the variant.

14. A method for producing cysteine or a derivative thereof, the method comprising:
a) culturing in a medium a microorganism comprising a MdtH variant, in which the amino acid corresponding to position 125 in the amino acid sequence of SEQ ID NO: 1 is substituted with another amino acid, or a polynucleotide encoding the variant, to thereby produce *O*-phosphoserine or a medium containing *O*-phosphoserine; and
b) reacting a sulfide with *O*-phosphoserine sulfhydrylase (OPSS) or a microorganism expressing OPSS and *O*-phosphoserine or the medium containing *O*-phosphoserine produced in step a).

15. Use of an MdtH variant for the production of OPS, cysteine, or a derivative of cysteine, wherein the amino acid corresponding to the 125^{th} position of the amino acid sequence of SEQ ID NO: 1 is substituted with another amino acid.

16. Use of an MdtH variant for the excretion of OPS from a microorganism, wherein the amino acid corresponding to the 125 ^{th} position of the amino acid sequence of SEQ ID NO: 1 is substituted with another amino acid.
